# EUROPEAN PATENT APPLICATION

(11) **EP 4 450 170 A1**
(43) Date of publication of application: **23.10.2024**
(21) Application number: 23745941.7
(22) Date of filing: 10.01.2023
(51) Int. Cl.: B05B 17/06, H03K 17/00

(54) **ULTRASONIC ATOMIZER**

(30) Priority: 26.01.2022 CN 202210096036
(71) Applicant: Shenzhen First Union Technology Co., Ltd., Shenzhen, Guangdong 518000 (CN)
(72) Inventor: LI, Xinjun, Shenzhen, Guangdong 518000 (CN); XU, Zhongli, Shenzhen, Guangdong 518000 (CN); LI, Yonghai, Shenzhen, Guangdong 518000 (CN)
(74) Representative: Ran, Handong
(86) International application number: PCT/CN2023/071618
(87) International publication number: WO 2023/143058

(57) **Abstract**

An ultrasonic atomizer, comprising: a liquid storage cavity (11), used for storing a liquid matrix; an ultrasonic atomization plate (12), used for generating oscillation to atomize the liquid matrix; a controller (13); a control circuit (14); and a power supply (15). The control circuit (14) comprises a power supply branch (141), a switch branch (142), a resonant branch (143), and an impedance branch (144); the resonant branch (143) is used for performing resonance in response to the on and off of the switch branch (142), so as to output a driving voltage according to a pulse voltage; and the impedance branch (144) is used for enabling the impedance of a combination of the impedance branch (144) and the ultrasonic atomization plate (12) to match the impedance of a combination of the power supply branch (141), the switch branch (142), and the resonant branch (143). The atomizer can improve the working efficiency.

## Description

### CROSS-REFERENCE TO RELATED APPLICATIONS

This application claims priority to Chinese Patent Application No. 202210096036.9, field with China National Intellectual Property Administration on January 26, 2022 and entitled "ULTRASONIC ATOMIZER", which is incorporated herein by reference in its entirety.

### TECHNICAL FIELD

This application relates to the field of ultrasonic atomizer technologies, and in particular, to an ultrasonic atomizer.

### BACKGROUND

In daily life, an ultrasonic atomizer may be used in a plurality of fields such as humidification, fragrance, sterilization, decoration, medical atomization, and electronic cigarettes.

The ultrasonic atomizer uses an ultrasonic atomization technology to achieve an atomization function. Specifically, in the ultrasonic atomizer, an ultrasonic atomization sheet can convert electrical energy to ultrasonic energy, and the ultrasonic energy can atomize water-soluble atomized liquid into tiny mist particles of 1 µm to 5 µm at normal temperature, so that the water-soluble atomized liquid can be atomized by using ultrasonic directional pressure by using water as a medium.

However, in conventional technologies, when the ultrasonic atomization sheet is driven, there is a lot of extra energy loss, resulting in low working efficiency of the ultrasonic atomizer.

### SUMMARY

Embodiments of this application are intended to provide an ultrasonic atomizer, to improve working efficiency of the ultrasonic atomizer.

According to a first aspect, this application provides an ultrasonic atomizer, including:
a liquid storage cavity, used for storing a liquid substrate;
an ultrasonic atomization sheet, used for generating oscillation to atomize the liquid substrate; and
a controller, a control circuit, and a power supply, where
the control circuit includes:
a power supply branch, connected to the power supply, and used for generating a direct current power supply based on the power supply;
a switching branch, connected to the controller and the power supply branch separately, and used for being on or off in response to a first pulse signal outputted by the controller, to generate a pulse voltage based on the direct current power supply;
a resonance branch, connected to the power supply branch and the switching branch separately, and used for resonating in response to on or off of the switching branch, to output, based on the pulse voltage, a driving voltage for driving the ultrasonic atomization sheet; and
an impedance branch, connected between the resonance branch and the ultrasonic atomization sheet, and used for enabling impedance of a combination of the impedance branch and the ultrasonic atomization sheet to match impedance of a combination of the power supply branch, the switching branch, and the resonance branch.

In an optional implementation, the power supply branch includes a first inductor; and
a first end of the first inductor is connected to the power supply, and a second end of the first inductor is connected to the switching branch and the resonance branch separately.

In an optional implementation, the switching branch includes a switching transistor; and

a first end of the switching transistor is connected to the controller, a second end of the switching transistor is connected to the ground, and a third end of the switching transistor is connected to the power supply branch and the resonance branch separately.

In an optional implementation, the switching branch further includes a first capacitor, a first end of the first capacitor is connected to the third end of the switching transistor, and a second end of the first capacitor is connected to the ground; and
the first capacitor is used for charging when the switching transistor is off and a current flowing through the resonance branch is less than a first current threshold, and the first capacitor is used for resonating with the resonance branch to discharge when the switching transistor is off and the current flowing through the resonance branch is greater than or equal to the first current threshold; where
when the first capacitor is discharged to a second current threshold, the switching transistor is on.

In an optional implementation, when the switching transistor is off, a frequency of a combination of the first capacitor and the resonance branch when resonating is lower than a frequency of the ultrasonic atomization sheet; and
when the switching transistor is on, a frequency of the resonance branch when resonating is higher than the frequency of the ultrasonic atomization sheet.

In an optional implementation, the frequency of the ultrasonic atomization sheet is any frequency between [2.9 MHZ-3.1 MHZ];
when the switching transistor is off, the frequency of the combination of the first capacitor and the resonance branch when resonating is any frequency between [2 MHZ-3 MHZ]; and
when the switching transistor is on, the frequency of the resonance branch when resonating is any frequency between [3.2 MHZ-4 MHZ].

In an optional implementation, the switching branch further includes a first resistor and a second resistor connected in series; and
a first end of a circuit formed by the first resistor and the second resistor connected in series is connected to the controller, a second end of the circuit formed by the first resistor and the second resistor connected in series is connected to the ground, and a connection point between the first resistor and the second resistor is connected to the first end of the switching transistor.

In an optional implementation, the resonance branch includes a second capacitor and a second inductor; and
a first end of the second capacitor is connected to the power supply branch and the switching branch separately, a second end of the second capacitor is connected to a first end of the second inductor, and a second end of the second inductor is connected to the impedance branch.

In an optional implementation, the resonance branch includes a sixth capacitor and a primary-side winding of a transformer; and
a first end of the sixth capacitor is connected to the power supply branch and the switching branch separately, a second end of the sixth capacitor is connected to a first end of the primary-side winding, and a second end of the primary-side winding is connected to the ground.

In an optional implementation, the impedance branch includes a sixth inductor and a secondary-side winding of the transformer; and
a first end of the sixth inductor is connected to a first end of the secondary-side winding of the transformer, a second end of the sixth inductor is connected to the ultrasonic atomization sheet, and a second end of the secondary-side winding of the transformer is connected to the ground.

In an optional implementation, the impedance branch includes a third capacitor; and
a first end of the third capacitor is connected to the resonance branch and the ultrasonic atomization sheet separately, and a second end of the third capacitor is connected to the ground.

In an optional implementation, the impedance branch further includes a third inductor; and
a first end of the third inductor is connected to the first end of the third capacitor and the resonance branch separately, and a second end of the third inductor is connected to the ultrasonic atomization sheet, or the first end of the third inductor is connected to the resonance branch, and the second end of the third inductor is connected to the first end of the third capacitor and the ultrasonic atomization sheet separately.

In an optional implementation, the impedance of the combination of the impedance branch and the ultrasonic atomization sheet includes an impedance real part and an impedance imaginary part, and when the impedance real part is equal to the impedance of the combination of the power supply branch, the switching branch, and the resonance branch, and the impedance imaginary part is zero, the impedance of the combination of the impedance branch and the ultrasonic atomization sheet matches the impedance of the combination of the power supply branch, the switching branch, and the resonance branch.

In an optional implementation, the control circuit further includes a driving branch;
the switching branch is connected to the controller through the driving branch, and the driving branch is connected to the power supply; and
the driving branch is used for receiving the first pulse signal, and outputting a second pulse signal to the switching branch based on the first pulse signal and the power supply, where a driving capability of the second pulse signal is stronger than that of the first pulse signal.

In an optional implementation, the driving branch includes a driving chip, and the driving chip includes a power input end, at least one signal input end, and at least one signal output end;
the power input end is connected to the power supply, the signal input end is connected to the controller, and the signal output end is connected to the switching branch, where
the signal input end is used for inputting the first pulse signal, and the signal output end is used for outputting the second pulse signal.

In an optional implementation, the control circuit further includes a current detection branch; and
the current detection branch is connected to the power supply, the power supply branch, and the controller separately, and the current detection branch is used for detecting a current flowing into the power supply branch.

In an optional implementation, the current detection branch includes an amplifier and a third resistor, the third resistor is connected to the amplifier, the power supply branch, and the power supply separately, and the amplifier is connected to the controller; and
the amplifier is configured to output a detection voltage based on a voltage across the third resistor, to enable the controller to determine, based on the detection voltage, the current flowing into the power supply branch.

The ultrasonic atomizer provided in this application may enable, based on on or off of the switching branch, the resonance branch to resonate during working of the ultrasonic atomization sheet. In this case, the resonance branch is purely resistive, which reduces reactive power of the resonance branch. In other words, a power loss is reduced, and the working efficiency of the ultrasonic atomizer is improved. Therefore, impedance of the resonance branch is minimum, a current is maximum, and an outputted driving voltage is maximum and is greater than a voltage of the power supply, so that a process of boosting the voltage of the power supply to drive the ultrasonic atomizer is implemented. In addition, the impedance of the combination of the impedance branch and the ultrasonic atomization sheet matches the impedance of the combination of the power supply branch, the switching branch, and the resonance branch through the impedance branch, to reduce reactive power of the combination of the impedance branch and the ultrasonic atomization sheet, so that the power loss can be further reduced, and the working efficiency of the ultrasonic atomizer can be improved.

### BRIEF DESCRIPTION OF THE DRAWINGS

One or more embodiments are exemplarily described with reference to the corresponding figures in the accompanying drawings, and the exemplary descriptions do not constitute limitations on embodiments. Elements in the accompanying drawings that have same reference numerals are represented as similar elements, and unless otherwise particularly stated, the figures in the accompanying drawings are not drawn to scale.
FIG. 1 is a schematic diagram of a structure of an ultrasonic atomizer according to an embodiment of this application;
FIG. 2 is a schematic diagram of a structure of an ultrasonic atomizer according to another embodiment of this application;
FIG. 3 is a schematic diagram of a structure of a control circuit according to an embodiment of this application;
FIG. 4 is a schematic diagram of a circuit structure of a control circuit according to an embodiment of this application;
FIG. 5 is a schematic diagram of a circuit structure of a control circuit according to another embodiment of this application;
FIG. 6 is a schematic diagram of a structure of a control circuit according to another embodiment of this application; and
FIG. 7 is a schematic diagram of a circuit structure of a control circuit according to still another embodiment of this application.

### DETAILED DESCRIPTION

To make the objectives, technical solutions, and advantages of embodiments of this application clearer, the technical solutions in embodiments of this application are clearly and completely described below with reference to the accompanying drawings. Apparently, the described embodiments are some rather than all embodiments of this application. All other embodiments obtained by a person of ordinary skill in the art based on embodiments of this application without making creative efforts shall fall within the protection scope of this application.

An ultrasonic atomizer is provided in embodiments of this application. The ultrasonic atomizer is provided with a resonance branch, to provide a driving voltage for an ultrasonic atomization sheet after a power supply of the power supply is boosted, so that reactive power is reduced and working efficiency is improved. In addition, an impedance matching process of the ultrasonic atomization sheet is implemented through an impedance branch, so that reactive power of the ultrasonic atomization sheet can be reduced, and the working efficiency can be further improved.

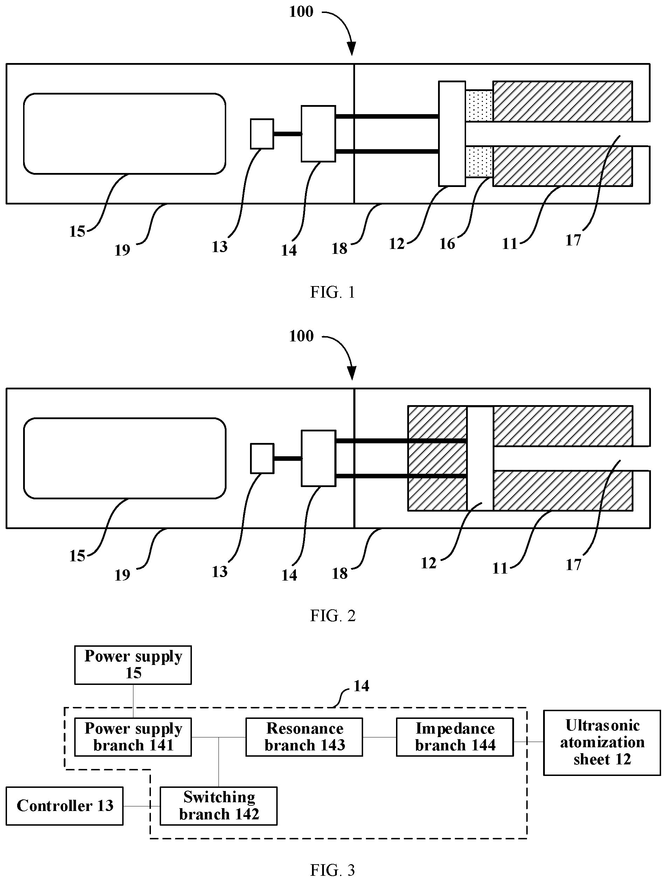

FIG. 1 is a schematic diagram of a structure of an ultrasonic atomizer according to an embodiment of this application. As shown in FIG. 1, an ultrasonic atomizer 100 includes a liquid storage cavity 11, an ultrasonic atomization sheet 12, a controller 13, a control circuit 14, and a power supply 15.

The liquid storage cavity 11 is used for storing a liquid substrate. The liquid substrate may include different substances according to different usage scenarios. For example, in a field of electronic cigarette atomization, the liquid substrate may include nicotine and/or fragrance and/or an aerosol generating substance (for example, glycerine), and in a field of medical atomization, the liquid substrate may include a drug for disease treatment or beneficial to health and/or solvent such as saline.

The ultrasonic atomization sheet 12 is in fluid communication with the liquid storage cavity 11. To be specific, the ultrasonic atomization sheet 12 may be directly disposed in the liquid storage cavity 11, or an atomization cavity in which the ultrasonic atomization sheet 12 is located may be directly communicated with the liquid storage cavity 11, or liquid may be transmitted between the ultrasonic atomization sheet 12 and the liquid storage cavity 11 by using a liquid absorption medium. The ultrasonic atomization sheet 12 is used for generating oscillation to atomize the liquid substrate, to be specific, to atomize, through oscillation, a liquid substrate transmitted to or near the ultrasonic atomization sheet 12 into an aerosol. Specifically, during use, the ultrasonic atomization sheet 12 breaks up the liquid substrate through high-frequency oscillation (where a preferred oscillation frequency is 1.7 MHz to 4.0 MHz, which exceeds a human hearing range and is in an ultrasonic frequency band) to produce an aerosol in which particles are naturally suspended.

The controller 13 may be a micro controller unit (Micro Controller Unit, MCU), a digital signal processing (Digital Signal Processing, DSP) controller, or the like. The controller 13 is electrically connected to the control circuit 14, and the controller 13 may be used for controlling at least one electronic element in the control circuit 14. The control circuit 14 is electrically connected to the ultrasonic atomization sheet 12, and the control circuit 14 is used for providing a driving voltage and a driving current for the ultrasonic atomization sheet 12 based on the power supply 15. In an implementation, the controller 13 and the control circuit 14 may be disposed on a printed circuit board (Printed Circuit Board, PCB).

The power supply 15 is used for supplying power. In an implementation, the power supply 15 is a battery. The battery may be a lithium-ion battery, a lithium metal battery, a lead-acid battery, a nickel-cadmium battery, a nickel-hydrogen battery, a lithium-sulfur battery, a lithium-air battery, a sodium-ion battery, or the like. This is not limited herein. In terms of scale, the battery in this embodiment of this application may be a cell, a battery module including a plurality of cells connected in series and/or connected in parallel, or the like. This is not limited herein. Certainly, in another embodiment, the battery may alternatively include more or fewer elements, or have different element configurations. This is not limited in embodiments of this application.

In an embodiment, the ultrasonic atomizer 100 further includes a liquid transmission medium 16, an air outlet channel 17, an upper housing 18, and a lower housing 19.

The liquid transmission element 16 is used for transmitting the liquid substrate between the liquid storage cavity 11 and the ultrasonic atomization sheet 12.

The air outlet channel 17 is used for outputting an inhalable vapor or aerosol produced from the liquid substrate for a user to inhale.

The upper housing 18 and the lower housing 19 are detachably connected. In an embodiment, the upper housing 18 and the lower housing 19 may be detachably connected in a buckle structure or a magnetic structure. The upper housing 18 and the lower housing 19 together serve to accommodate and protect other elements and components. The liquid storage cavity 11, the ultrasonic atomization sheet 12, the liquid transmission element 16, and the air outlet channel 17 are all disposed in the upper housing 18, and the controller 13, the control circuit 14, and the power supply 15 are all disposed in the lower housing 19.

The upper housing 18 is detachably aligned with the lower housing 19 in a functional relationship. Various mechanisms may be used to connect the lower housing 19 to the upper housing 18, to create a threaded engagement, a press fit engagement, an interference fit, a magnetic engagement, and the like. In some implementations, when the upper housing 18 and the lower housing 19 are in an assembled configuration, the ultrasonic atomizer 100 may be basically rod-shaped, flat-cylindrical, rod-shaped, column-shaped, or the like.

The upper housing 18 and the lower housing 19 may be made of any suitable material having a structure in good condition. In some examples, the upper housing 18 and the lower housing 19 may be made of a metal or alloy such as stainless steel or aluminum. Other suitable materials include various plastics (such as polycarbonate), metal-plating over plastic (metal-plating over plastic), ceramics, and the like.

It should be noted that, a hardware structure of the ultrasonic atomizer 100 shown in FIG. 1 is merely an example, and the ultrasonic atomizer 100 may have more or fewer components than those shown in the figure, may combine two or more components, or may have a different component configuration. The components shown in the figure may be implemented by hardware, software, or a combination of hardware and software including one or more signal processing and/or application-specific integrated circuits. For example, as shown in FIG. 2, the ultrasonic atomization sheet 12 may be disposed in the liquid storage cavity 11, so that the use of the liquid transmission element 16 can be reduced, which is beneficial to costs saving.

In addition, it may be understood that the ultrasonic atomizer 100 shown in FIG. 1 or FIG. 2 may be used in a variety of different occasions and plays different roles. This is not specifically limited in embodiments of this application. For example, in an embodiment, the ultrasonic atomizer 100 is used in a medical field. In this case, the ultrasonic atomizer 100 may be a medical atomizer. The medical atomizer atomizes liquid medicine added therein for a patient to inhale, to achieve an effect of auxiliary treatment. For example, in another embodiment, the ultrasonic atomizer 100 may alternatively be used as an electronic product, such as an electronic cigarette. The electronic cigarette is an electronic product that converts a nicotine solution into an aerosol through atomization for a user to inhale.

FIG. 3 is a schematic diagram of a circuit structure of an ultrasonic atomizer according to an embodiment of this application. As shown in FIG. 3, a control circuit 14 includes a power supply branch 141, a switching branch 142, a resonance branch 143, and an impedance branch 144.

The power supply branch 141 is connected to a power supply 15, the switching branch 142 is connected to a controller 12 and the power supply branch 141 separately, the resonance branch 143 is connected to the power supply branch 141 and the switching branch 142 separately, and the impedance branch 144 is connected between the resonance branch 143 and an ultrasonic atomization sheet 12. Specifically, a first end of the power supply branch 141 is connected to the power supply 15, a second end of the power supply branch 141 is connected to a first end of the switching branch 142 and a first end of the resonance branch 143 separately, a second end of the switching branch 142 is connected to the controller 13, a second end of the resonance branch 143 is connected to a first end of the impedance branch 144, and a second end of the impedance branch 144 is connected to a first end of the ultrasonic atomization sheet 12.

Specifically, the power supply branch 141 is used for generating a direct current power supply based on the power supply 15. The switching branch 142 is used for being on or off in response to a first pulse signal outputted by the controller 13, to generate a pulse voltage based on the direct current power supply. The resonance branch 143 is used for resonating in response to on or off of the switching branch 142, to output, based on the pulse voltage, a driving voltage for driving the ultrasonic atomization sheet 12. The impedance branch 144 is used for enabling impedance of a combination of the impedance branch 144 and the ultrasonic atomization sheet 12 to match impedance of a combination of the power supply branch 141, the switching branch 142, and the resonance branch 143.

In this embodiment, when the ultrasonic atomization sheet 12 needs to be driven, first, the power supply 15 is converted into the direct current power supply to be outputted after passing through the power supply branch 141. In this case, the controller 13 outputs the first pulse signal to control the switching branch 142 to continuously switch between being on and being off, so that the direct current power supply outputted by the power supply branch 141 is converted into an alternating current power supply, that is, the pulse voltage. Then, after resonance, the resonance branch 143 can boost the received pulse voltage and use a boosted driving voltage to drive the ultrasonic atomization sheet 12. Because the resonance branch 143 implements resonance, the resonance branch 143 is substantially purely resistive, and reactive power of the resonance branch 143 can be reduced. In other words, a power loss is reduced, so that working efficiency of the ultrasonic atomizer 100 is improved. In addition, in this case, impedance of the resonance branch 143 is minimum and a current is maximum, and a great driving voltage may be output to drive the ultrasonic atomization sheet 12 to operate stably.

Moreover, the ultrasonic atomization sheet 12 may be equivalent to a capacitive load. After the resonance branch 143 resonates, the combination of the power supply branch 141, the switching branch 142, and the resonance branch 143 is a purely resistive output. If energy is directly transmitted between the two (namely, the capacitive load and the purely resistive output), a large amount of reactive power may be generated, resulting in great reducing of efficiency of driving the ultrasonic atomization sheet 12.

Therefore, in this embodiment, the impedance branch 144 is provided to enable the impedance of the combination of the impedance branch 144 and the ultrasonic atomization sheet 12 to match the impedance of the combination of the power supply branch 141, the switching branch 142, and the resonance branch 143. In this way, reactive power of the combination of the impedance branch 144 and the ultrasonic atomization sheet 12 can be reduced, to reduce a power loss. The ultrasonic atomization sheet 12 may obtain great driving energy, so that the efficiency of driving the ultrasonic atomization sheet 12 is improved, and the working efficiency of the ultrasonic atomizer 100 is further improved.

Specifically, in an implementation, the impedance (Zh) of the combination of the impedance branch 144 and the ultrasonic atomization sheet 12 includes an impedance real part (Rh) and an impedance imaginary part (j *Xh). When the impedance real part is equal to the impedance (Z0) of the combination of the power supply branch 141, the switching branch 142, and the resonance branch 143, and the impedance imaginary part is zero, the impedance of the combination of the impedance branch 144 and the ultrasonic atomization sheet 12 matches the impedance of the combination of the power supply branch 141, the switching branch 142, and the resonance branch 143.

Zh = Rh + j *Xh. In addition, because the impedance of the combination of the power supply branch 141, the switching branch 142, and the resonance branch 143 is purely resistive, Z0 = R0. R0 represents resistance of a combination of the switching branch 142 and the resonance branch 143. Therefore, if the impedance of the combination of the impedance branch 144 and the ultrasonic atomization sheet 12 matches the impedance of the combination of the power supply branch 141, the switching branch 142, and the resonance branch 143, conditions to be satisfied are: Rh = R0, and j *Xh = 0. In this case, working efficiency of the ultrasonic atomization sheet 12 is high.

In different application cases, there are a plurality of manners to satisfy the foregoing conditions. This is not specifically limited in this application. For example, in an embodiment, first, a suitable load is configured in the resistance branch 144 to implement that Rh = R0. In this case, the ultrasonic atomization sheet 12 may obtain maximum driving power. Then, an impedance opposite to capacitive reactance of the ultrasonic atomization sheet 12 needs to be further configured in the resistance branch 144, to offset the capacitive reactance of the ultrasonic atomization sheet 12 and eliminate reactive power caused by the capacitive reactance.

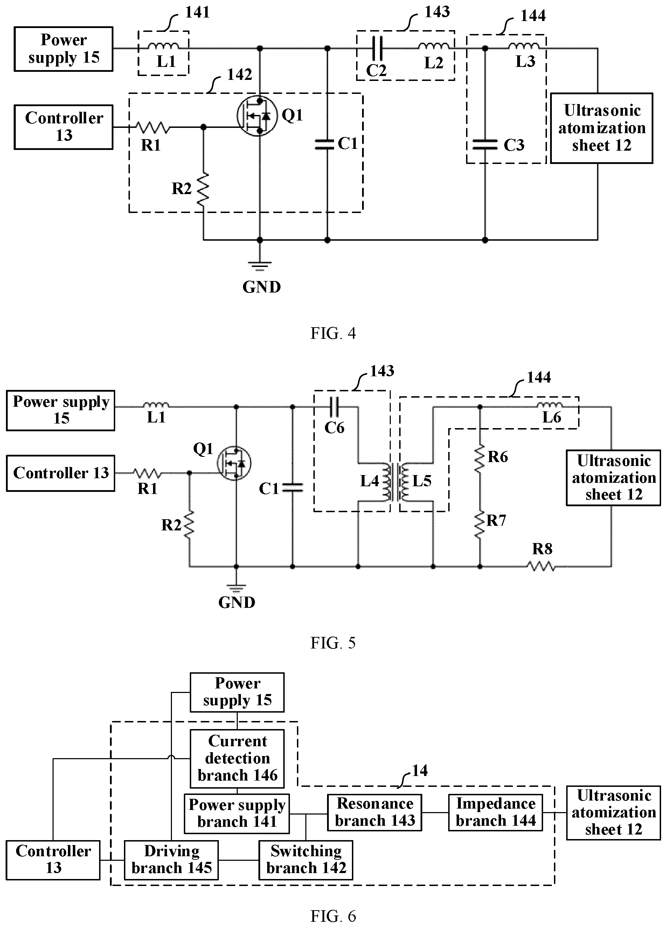

In an embodiment, as shown in FIG. 4, the power supply branch 141 includes a first inductor L1. A first end of the first inductor L1 is connected to the power supply 15, and a second end of the first inductor L1 is connected to the switching branch 142 and the resonance branch 143 separately.

Specifically, the first inductor L1 is a high-frequency choke, which only has a great blocking effect on a high-frequency alternating current, and has a less blocking effect on a low-frequency alternating current, and an even less blocking effect on a direct current. Therefore, the high-frequency choke may be used for "passing a direct current, blocking an alternating current, passing low frequency, and blocking high frequency". Therefore, the first inductor L1 may allow the direct current to pass through to provide energy for subsequent circuits, in other words, to implement a process of outputting the direct current power supply based on the power supply 15. In addition, the first inductor L1 may be further used for preventing a high-frequency short circuit.

For example, FIG. 4 also shows a structure of the switching branch 142. As shown in FIG. 4, the switching branch 142 includes a switching transistor Q1. A first end of the switching transistor Q1 is connected to the controller 13, a second end of the switching transistor Q1 is connected to the ground GND, and a third end of the switching transistor Q1 is connected to the power supply branch 141 and the resonance branch 143 separately.

In this embodiment, for example, the switching transistor Q1 is an N-channel metal-oxide-semiconductor field-effect transistor (namely, an NMOS transistor). Specifically, a gate of the NMOS transistor is the first end of the switching transistor Q1, a source of the NMOS transistor is the second end of the switching transistor Q1, and a drain of the NMOS transistor is the third end of the switching transistor Q1.

In addition, in other embodiments, the switching transistor Q1 may alternatively be a positive-channel metal-oxide-semiconductor field-effect transistor or a signal relay. The switching transistor Q1 may alternatively be at least one of a triode, an insulated gate bipolar transistor, an integrated gate commutated thyristor, a gate turn-off thyristor, a junction gate field effect transistor, a MOS controlled thyristor, a gallium nitride-based power device, a silicon carbide-based power device, or a thyristor.

In an embodiment, the switching branch 142 further includes a first resistor R1 and a second resistor R2 connected in series. A first end of a circuit formed by the first resistor R1 and the second resistor R2 connected in series is connected to the controller 13, a second end of the circuit formed by the first resistor R1 and the second resistor R2 connected in series is connected to the ground GND, and a connection point between the first resistor R1 and the second resistor R2 is connected to the first end of the switching transistor Q1.

In this embodiment, the first resistor R1 and the second resistor R2 are used for dividing a voltage of the first pulse signal output by the controller 13, to obtain a voltage of the first end of the switching transistor Q1. When a divided voltage on the second resistor R2 is greater than an on-state voltage of the switching transistor Q1, the switching transistor Q1 is on, otherwise, the switching transistor Q1 is off.

In an embodiment, the switching branch 142 further includes a first capacitor C1. A first end of the first capacitor C1 is connected to the third end of the switching transistor Q1, and a second end of the first capacitor C1 is connected to the ground GND.

Specifically, the first capacitor C1 is used for charging when the switching transistor Q1 is off and a current flowing through the resonance branch 143 is less than a first current threshold, and the first capacitor C1 is used for resonating with the resonance branch 143 to discharge when the switching transistor Q 1 is off and the current flowing through the resonance branch 143 is greater than or equal to the first current threshold. When the first capacitor C 1 is discharged to a second current threshold, the switching transistor Q1 is on.

It may be understood that values of the first current threshold and the second current threshold are both related to parameters of the first capacitor C1 and the resonance branch 143. In other words, in different application scenarios, different first current thresholds and second current thresholds may be obtained by selecting different first capacitors C1 and resonance branches 143. This is not specifically limited in this embodiment of this application.

In this embodiment, the first capacitor C1 may play a role of voltage hysteresis. Specifically, at a moment when the switching transistor Q1 is off, a voltage between the second end and the third end of the switching transistor Q1 does not increase suddenly, but first maintains a voltage across the first capacitor C1. After a current between the second end and the third end of the switching transistor Q1 drops to zero, the voltage between the second end and the third end of the switching transistor Q1 starts to increase. Therefore, soft turn-off of the switching transistor Q1 is achieved.

In this case, the current flowing through the resonance branch 143 is less than the first current threshold, and the first capacitor C1 is charged. Then, the current of the resonance branch 143 gradually increases until the current is greater than or equal to the first current threshold. In this case, the current of the resonance branch 143 is greater than a current on the first inductor L1, and the first capacitor C1 resonates with the resonance branch 143 to discharge. Next, when the first capacitor C1 is discharged to the second current threshold, the switching transistor Q1 is on. It can be learned that, suitable first capacitor C1 and resonance branch 143 are selected to enable the second current threshold to be zero, so that zero voltage turn-on of the switching transistor Q1 can be achieved. In other words, soft turn-on of the switching transistor Q1 is achieved.

It may be understood that when a transistor (such as the switching transistor Q1) is in an on/off state, 100% efficiency can be achieved theoretically. However, due to an impact of transistor barrier capacitance, diffusion capacitance, and distributed capacitance in the circuit, specific transition time is required for the transistor to switch from saturation to cutoff or from cutoff to saturation. As a result, a collector current and a collector voltage of the transistor may be great during the transition time, resulting in increased transistor power consumption. Usually, when parasitic capacitance is not too large and a working frequency is low, the impact of the increased transistor power consumption may be ignored. However, when the working frequency is high, the increased transistor power consumption cannot be ignored, which reduces efficiency and even damages a device.

Therefore, in this embodiment, the first capacitor C1 and the resonance branch 143 are provided to implement a soft-switching process (including the soft turn-on and the soft turn-off) of the switching transistor Q1, in other words, to keep a product of the voltage and the current always zero when the switching transistor Q1 is on or off. Therefore, a switching loss of the switching transistor Q1 is close to zero, and switching efficiency of the switching transistor Q1 is high, so that the working efficiency of the ultrasonic atomizer 100 is further improved.

Then, in an embodiment, to ensure that the switching transistor Q1 can work in a soft-switching state, the following parameters may be configured. First, when the switching transistor Q1 is off, a frequency (denoted as a first resonance frequency) of a combination of the first capacitor C1 and the resonance branch 143 when resonating is configured to be lower than a frequency of the ultrasonic atomization sheet 12. In addition, when the switching transistor Q1 is on, a frequency (denoted as a second resonance frequency) of the resonance branch 143 when resonating is configured to be higher than the frequency of the ultrasonic atomization sheet 12.

The first resonance frequency and the second resonance frequency may be configured based on an actual selected ultrasonic atomization sheet 12. This is not specifically limited in this embodiment of this application.

For example, in an optional implementation, a frequency of the selected ultrasonic atomization sheet 12 is any frequency between [2.9 MHz-3.1 MHz], so that the first resonance frequency may be any frequency between [2 MHz-3 MHz], and the second resonance frequency may be any frequency between [3.2 MHz-4 MHz]. For example, the frequency of the ultrasonic atomization sheet is 3 MHz, the first resonance frequency is 2.5 MHz, and the second resonance frequency is 4 MHz, so that the first resonance frequency is lower than the frequency of the ultrasonic atomization sheet, and the frequency of the ultrasonic atomization sheet is lower than the second resonance frequency, thereby achieving the soft-switching of the switching transistor Q1 and improving the switching efficiency of the switching transistor Q1.

For another example, in another optional implementation, the frequency of the selected ultrasonic atomization sheet 12 is any frequency between [10 KHz-10 MHz], so that the first resonance frequency and the second resonance frequency may be configured based on the frequency of the actual used ultrasonic atomization sheet 12, provided that the first resonance frequency is lower than the frequency of the ultrasonic atomization sheet and the frequency of the ultrasonic atomization sheet is lower than the second resonance frequency. For example, if the frequency of the actual used ultrasonic atomization sheet 12 is 2.4 MHz or 2,7 MHz, the first resonance frequency may be any frequency between [1.5 MHz-2 MHz], and the second resonance frequency may be any frequency between [3 MHz-3.5 MHz]. If the frequency of the actual used ultrasonic atomization sheet 12 is 130 KHz or 160 KHz, the first resonance frequency may be any frequency between [100 KHz-120 KHz], and the second resonance frequency may be any frequency between [180 KHz-200 KHz],

For example, FIG. 4 also shows a structure of the resonance branch 143. As shown in FIG. 4, the resonance branch 143 includes a second capacitor C2 and a second inductor L2. A first end of the second capacitor C2 is connected to the power supply branch 141 (that is, the second end of the first inductor L1) and the switching branch 142 (that is, the third end of the switching transistor Q1) separately, a second end of the second capacitor C2 is connected to a first end of the second inductor L2, and a second end of the second inductor L2 is connected to the impedance branch 144.

In this embodiment, when the second capacitor C2 and the second inductor L2 form series resonance, a circuit formed by the second capacitor C2 and the second inductor L2 is purely resistive. In this case, impedance is minimum, a current is maximum, and a high voltage N times greater than the pulse voltage input to the resonance branch 143 may be generated on the second capacitor C2 and the second inductor L2. N is greater than 1. The high voltage is the driving voltage for driving the ultrasonic atomization sheet 12. Then, the ultrasonic atomization sheet 12 may obtain sufficient driving energy, which is beneficial to maintaining a stable operation of the ultrasonic atomization sheet 12.

For example, FIG. 5 also shows another structure of the resonance branch 143. As shown in FIG. 5, the resonance branch 143 includes a sixth capacitor C6 and a primary-side winding L4 of a transformer. A first end of the sixth capacitor C6 is connected to the power supply branch 141 and the switching branch 142 separately, a second end of the sixth capacitor C6 is connected to a first end of the primary-side winding L4, and a second end of the primary-side winding L4 is connected to the ground GND.

In this embodiment, resonance formed by the sixth capacitor C6 and the primary-side winding L4 of the transformer is similar to the resonance formed by the second capacitor C2 and the second inductor L2. This is within the scope that can be easily understood by a person skilled in the art and is not described in detail herein again.

In an embodiment, as shown in FIG. 4, the impedance branch 144 includes a third capacitor C3 and a third inductor L3. A first end of the third inductor L3 is connected to a first end of the third capacitor C3 and the resonance branch 143 separately, and a second end of the third inductor L3 is connected to the ultrasonic atomization sheet 12.

It should be noted that, FIG. 4 only shows an example of a structure of the impedance branch 144. In another embodiment, the impedance branch 144 may alternatively be in another structure. This is not specifically limited in embodiments of this application, provided that the impedance of the combination of the impedance branch 144 and the ultrasonic atomization sheet 12 matches the impedance of the combination of the power supply branch 141, the switching branch 142, and the resonance branch 143.

For example, in an implementation, the impedance branch 144 may only include the third capacitor C3. In this case, the first end of the third capacitor C3 is connected to the resonance branch 143 and the ultrasonic atomization sheet 12 separately, and a second end of the third capacitor C3 is connected to the ground GND.

For another example, in another implementation, the impedance branch 144 still includes the third capacitor C3 and the third inductor L3. In this implementation, the first end of the third inductor L3 is connected to the resonance branch 143, and the second end of the third inductor L3 is connected to the first end of the third capacitor C3 and the ultrasonic atomization sheet 12 separately.

For another example, in still another implementation, as shown in FIG. 5, the impedance branch includes a sixth inductor L6 and a secondary-side winding L5 of the transformer. A first end of the sixth inductor L6 is connected to a first end of the secondary-side winding L5 of the transformer, a second end of the sixth inductor L6 is connected to the ultrasonic atomization sheet 12, and a second end of the secondary-side winding L5 of the transformer is connected to the ground GND.

In an embodiment, continue to refer to FIG. 5. The control circuit 14 further includes a sixth resistor R6, a seventh resistor R7, and an eighth resistor R8. A first end of the sixth resistor R6 is connected to the first end of the sixth inductor L6, a second end of the sixth resistor R6 is connected to a first end of the seventh resistor R7, a second end of the seventh resistor R7 and a first end of the eighth resistor R8 are both connected to the ground GND, and a second end of the eighth resistor R8 is connected to the ultrasonic atomization sheet 12.

In this embodiment, the sixth resistor R6 and the seventh resistor R7 are used for implementing a voltage detection function, and the eighth resistor R8 is used for implementing a current detection function.

The following describes a working principle of the circuit structure shown in FIG. 4.

Before the switching transistor Q1 is off, the switching transistor Q1 is in an on state, and the power supply 15 forms a loop through the first inductor L1, the switching transistor Q1, and the ground GND, to store energy from the power supply 15 in the first inductor L1. The first inductor L1 has a great inductance value and stores a large amount of energy, and may be equivalent to a constant current source.

Then, the first pulse signal outputted by the controller 13 is in a low-level state, the switching transistor Q1 is off, a current originally flowing through the switching transistor Q1 is transferred to the first capacitor C1, and a current in the switching transistor Q1 is zero. The power supply 15, the first inductor L 1, and the first capacitor C1 form a loop. The power supply 15 starts to charge the first capacitor C1, and a voltage across the first capacitor C1 gradually increases. In this case, a current on the resonance branch 143 gradually decreases from a negative axis to a zero axis.

When the current on the first inductor L1 is equal to a current on the first capacitor C1, currents on the resonance branch 143 and the ultrasonic atomization sheet 12 are zero. The currents on the resonance branch 143 and the ultrasonic atomization sheet 12 change from negative to positive and increase gradually. When the current on the resonance branch 143 is equal to the current on the first inductor L1, a current flowing through the first capacitor C1 is zero. In this case, the voltage across the first capacitor C1 reaches a maximum value.

As the current on the resonance branch 143 further increases and is greater than the current on the first inductor L1, the first capacitor C1 begins to be discharged. The voltages of both ends of the first capacitor C1 gradually decrease. When charge stored in the first capacitor C1 is completely discharged, the first pulse signal outputted by the controller 13 switches from the low-level state to a high-level state, and the switching transistor Q1 is on. It can be learned that, when the switching transistor Q1 is on, a voltage (that is, the voltage across the first capacitor C1) between the second end and the third end of the switching transistor Q1 is zero. Therefore, no loss is generated when the switching transistor Q1 is on.

Then, after the switching transistor Q1 is turned on, the first capacitor C1 is shortcircuited, and the voltage across the first capacitor C1 is zero. In this case, an initial current flowing through the switching transistor Q1 is zero and begins to gradually increase, and the current on the resonance branch 143 gradually decreases. When the current flowing through the switching transistor Q1 is equal to the current on the first inductor L1, the current on the resonance branch 143 is zero.

The current on the resonance branch 143 changes from zero to a negative value, a current amplitude gradually increases, and the current flowing through the switching transistor Q1 maintains an increasing stage. The power supply 15 stores the energy in the first inductor L1 again until the first pulse signal outputted by the controller 13 switches from the high-level state to the low-level state again, and the switching transistor Q1 is off again.

The foregoing process is repeated and executed in a loop to implement the driving process for the ultrasonic atomization sheet 12. In addition, in the process, the first capacitor C1, the second capacitor C2, and the first inductor L2 are used in conjunction to implement the soft-switching process of the switching transistor Q1, and there is a little damage to the switching transistor Q1, so that the working efficiency of the ultrasonic atomizer 100 is high. Moreover, the impedance of the combination of the impedance branch 144 and the ultrasonic atomization sheet 12 matches the impedance of the combination of the power supply branch 141, the switching branch 142, and the resonance branch 143 through the impedance branch 144, to reduce reactive power generated by the ultrasonic atomization sheet 12, and the driving efficiency for the ultrasonic atomization sheet 12 is high, so that the working efficiency of the ultrasonic atomizer 100 is further improved.

In an embodiment, as shown in FIG. 6, the control circuit 14 further includes a driving branch 145. The switching branch 142 is connected to the controller 13 through the driving branch 145, and the driving branch 145 is connected to the power supply 15. Specifically, the second end of the switching branch 142 is connected to a first end of the driving branch 145, a second end of the driving branch 145 is connected to the controller 13, and a third end of the driving branch 145 is connected to the power supply 15.

Specifically, the driving branch 145 is used for receiving the first pulse signal outputted by the controller 13, and outputting a second pulse signal to the switching branch 142 based on the first pulse signal and the power supply 15. A driving capability of the second pulse signal is stronger than that of the first pulse signal. The driving branch 145 is used for enhancing the first pulse signal outputted by the controller 13, and then outputting the second pulse signal, to more efficiently drive the switching transistor Q1 in the switching branch 142 to turn on or off quickly.

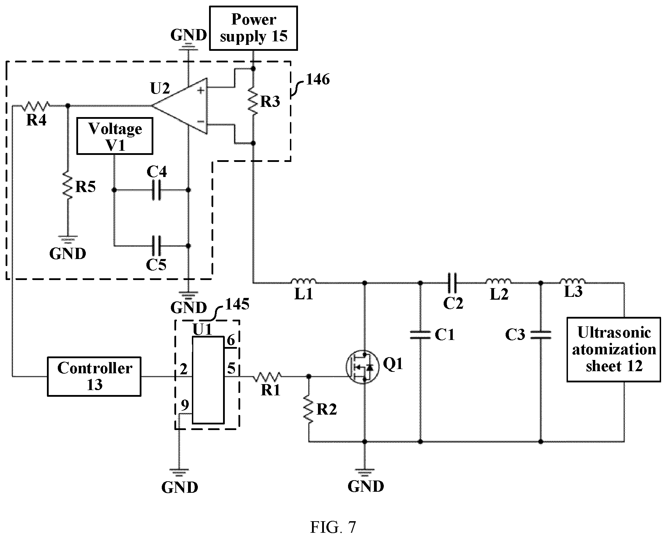

For example, FIG. 7 shows a structure of the driving branch 145. As shown in FIG. 7, the driving branch 145 includes a driving chip U1. The driving chip U1 includes a power input end, at least one signal input end, and at least one signal output end. In this embodiment, the power input end is a sixth pin of the driving chip U1. The at least one signal input end includes a signal input end, which is a second pin of the driving chip U1. The at least one signal output end includes a signal output end, which is a fifth pin of the driving chip U1.

The power input end is connected to the power supply 15, the signal input end is connected to the controller 13, and the signal output end is connected to the switching branch 142. The signal input end is used for inputting the first pulse signal, and the signal output end is used for outputting the second pulse signal.

Specifically, the sixth pin of the driving chip U1 is used for connecting to the power supply 15. The second pin of the driving chip U1 is connected to the controller 13. The fifth pin of the driving chip U1 is connected to the switching branch 142. The second pin of the driving chip U1 is used for inputting the first pulse signal, and the fifth pin of the driving chip U1 is used for outputting the second pulse signal.

In this embodiment, the driving chip U1 is provided to improve a driving capability of a pulse signal outputted by the controller 13. Therefore, the switching sub-branch 142 can be driven quickly to maintain the stable operation of the ultrasonic atomization sheet 12. In addition, a greater current inputted to the sixth pin of the driving chip U1 indicates a stronger driving capability outputted by the fifth pin of the driving chip U1.

In an embodiment, the driving chip U1 may be an integrated chip of model SGM48000. Certainly, in another embodiment, an integrated chip of another model may alternatively be used. This is not limited in embodiments of this application. In addition, because there are different types of the driving chip, when driving chips of other types are used, specific pin definitions may be different, but functions and signal definitions are the same. If the driving chips of other types are used, configuration may be performed in a similar manner to the foregoing embodiment. This is within the scope that can be easily understood by a person skilled in the art and is not described in detail herein again.

In addition, in this embodiment, for example, the power supply 15 is an input power supply for the driving chip U1. In other words, in this embodiment, the power supply 15 is used as a power supply for both the driving chip U1 and the ultrasonic atomization sheet 12, to achieve an objective of costs saving. In another embodiment, to prevent the driving chip U1 and the ultrasonic atomization sheet 12 from interfering with each other during working, two different power supplies may be used for respectively supplying power to the driving chip U1 and the ultrasonic atomization sheet 12, to improve working stability of both the driving chip U1 and the ultrasonic atomization sheet 12.

In an embodiment, as shown in FIG. 6, the control circuit 14 further includes a current detection branch 146. The current detection branch 146 is connected to the power supply 15, the power supply branch 141, and the controller 13 separately. Specifically, a first end of the current detection branch 146 is connected to the power supply 15, a second end of the current detection branch 146 is connected to the power supply branch 141, and a third end of the current detection branch 146 is connected to the controller 13.

In this embodiment, the current detection branch 146 is used for detecting a current flowing into the power supply branch 141. Then, the controller 13 may determine, based on the current, whether the ultrasonic atomization sheet 12 has an abnormality such as excessive current during working, so that the controller 13 may deal with the abnormality timely, which is beneficial to reducing a risk of the ultrasonic atomization sheet 12 being damaged.

For example, FIG. 7 shows a structure of the current detection branch 146. As shown in FIG. 7, the current detection branch 146 includes an amplifier U2 and a third resistor R3. The third resistor R3 is connected to the amplifier U2 and the power supply branch 141 separately, and the amplifier U2 is connected to the controller 13.

Specifically, a first end of the third resistor R3 is connected to the power supply 15 and a non-inverting input end of the amplifier U2 separately, a second end of the third resistor R3 is connected to an inverting input end of the amplifier U2 and the first end of the first inductor L1 separately, an output end of the amplifier U2 is connected to the controller 13, a ground end of the amplifier U2 is connected to the ground GND, and a power supply end of the amplifier U2 is connected to a voltage V1.

In this embodiment, the amplifier U2 is configured to output a detection voltage based on a voltage across the third resistor R3, to enable the controller 13 to determine, based on the detection voltage, a current flowing into the power supply branch 141. Specifically, the amplifier U2 may amplify the received voltage across the third resistor R3 by K times and then output the detection voltage. K is a positive integer. Then, after obtaining the detection voltage, the controller 13 may determine, based on a relationship between the detection voltage and the current flowing into the power supply branch 141, the current flowing into the power supply branch 141.

In an embodiment, the current detection branch 146 further includes a fourth capacitor C4, a fifth capacitor C5, a fourth resistor R4, and a fifth resistor R5. The fourth capacitor C4 and the fifth capacitor C5 are filter capacitors, the fifth resistor R5 is a current limiting resistor, and the fourth resistor R4 is a pull-down resistor.

It should be noted that, in embodiments shown in the foregoing figures, the resistor is presented as a single resistor, and the capacitor is presented as a single capacitor. In another embodiment, the resistor may alternatively be an integration of series, parallel, or mixed resistors, and the capacitor may alternatively be an integration of series, parallel, or mixed capacitors.

The connection described in this application may be a direct connection, to be specific, a connection between two components, or an indirect connection, to be specific, an indirect connection between two components may be formed by using one or more elements.

Finally, it should be noted that: the foregoing embodiments are merely intended for describing the technical solutions of this application, but not for limiting this application. Under the ideas of this application, the technical features in the foregoing embodiments or different embodiments may also be combined, the steps may be performed in any order, and many other changes of different aspects of this application also exists as described above, and these changes are not provided in detail for simplicity. Although this application is described in detail with reference to the foregoing embodiments, a person of ordinary skill in the art should understand that: modifications may still be made to the technical solutions described in the foregoing embodiments, or equivalent replacements may be made to the part of the technical features; and these modifications or replacements will not cause the essence of corresponding technical solutions to depart from the scope of the technical solutions in embodiments of this application.

## Claims

1. An ultrasonic atomizer, comprising:
a liquid storage cavity, used for storing a liquid substrate;
an ultrasonic atomization sheet, used for generating oscillation to atomize the liquid substrate; and
a controller, a control circuit, and a power supply,
wherein the control circuit comprises:
a power supply branch, connected to the power supply, and used for generating a direct current power supply based on the power supply;
a switching branch, connected to the controller and the power supply branch separately, and used for being on or off in response to a first pulse signal outputted by the controller, to generate a pulse voltage based on the direct current power supply;
a resonance branch, connected to the power supply branch and the switching branch separately, and used for resonating in response to on or off of the switching branch, to output, based on the pulse voltage, a driving voltage for driving the ultrasonic atomization sheet; and
an impedance branch, connected between the resonance branch and the ultrasonic atomization sheet, and used for enabling impedance of a combination of the impedance branch and the ultrasonic atomization sheet to match impedance of a combination of the power supply branch, the switching branch, and the resonance branch.

2. The ultrasonic atomizer according to claim 1, wherein:
the power supply branch comprises a first inductor;
a first end of the first inductor is connected to the power supply; and
a second end of the first inductor is connected to the switching branch and the resonance branch separately.

3. The ultrasonic atomizer according to claim 1, wherein:
the switching branch comprises a switching transistor;
a first end of the switching transistor is connected to the controller;
a second end of the switching transistor is connected to the ground; and
a third end of the switching transistor is connected to the power supply branch and the resonance branch separately.

4. The ultrasonic atomizer according to claim 3, wherein:
the switching branch further comprises a first capacitor;
a first end of the first capacitor is connected to the third end of the switching transistor;
a second end of the first capacitor is connected to the ground;
the first capacitor is used for:
charging when the switching transistor is off and a current flowing through the resonance branch is less than a first current threshold, and
resonating with the resonance branch to discharge when the switching transistor is off and the current flowing through the resonance branch is greater than or equal to the first current threshold; and
when the first capacitor is discharged to a second current threshold, the switching transistor is on.

5. The ultrasonic atomizer according to claim 4, wherein:
when the switching transistor is off, a frequency of a combination of the first capacitor and the resonance branch when resonating is lower than a frequency of the ultrasonic atomization sheet; and
when the switching transistor is on, a frequency of the resonance branch when resonating is higher than the frequency of the ultrasonic atomization sheet.

6. The ultrasonic atomizer according to claim 5, wherein:
the frequency of the ultrasonic atomization sheet is any frequency between [2.9 MHZ-3.1 MHZ];
when the switching transistor is off, the frequency of the combination of the first capacitor and the resonance branch when resonating is any frequency between [2 MHZ-3 MHZ]; and
when the switching transistor is on, the frequency of the resonance branch when resonating is any frequency between [3.2 MHZ-4 MHZ].

7. The ultrasonic atomizer according to claim 3, wherein:
the switching branch further comprises a first resistor and a second resistor connected in series;
a first end of a circuit formed by the first resistor and the second resistor connected in series is connected to the controller;
a second end of the circuit formed by the first resistor and the second resistor connected in series is connected to the ground; and
a connection point between the first resistor and the second resistor is connected to the first end of the switching transistor.

8. The ultrasonic atomizer according to claim 1, wherein:
the resonance branch comprises a second capacitor and a second inductor; and
a first end of the second capacitor is connected to the power supply branch and the switching branch separately;
a second end of the second capacitor is connected to a first end of the second inductor; and
a second end of the second inductor is connected to the impedance branch.

9. The ultrasonic atomizer according to claim 1, wherein:
the resonance branch comprises a sixth capacitor and a primary-side winding of a transformer;
a first end of the sixth capacitor is connected to the power supply branch and the switching branch separately;
a second end of the sixth capacitor is connected to a first end of the primary-side winding; and
a second end of the primary-side winding is connected to the ground.

10. The ultrasonic atomizer according to claim 9, wherein:
the impedance branch comprises a sixth inductor and a secondary-side winding of the transformer;
a first end of the sixth inductor is connected to a first end of the secondary-side winding of the transformer;
a second end of the sixth inductor is connected to the ultrasonic atomization sheet; and
a second end of the secondary-side winding of the transformer is connected to the ground.

11. The ultrasonic atomizer according to claim 1, wherein:
the impedance branch comprises a third capacitor;
a first end of the third capacitor is connected to the resonance branch and the ultrasonic atomization sheet separately; and
a second end of the third capacitor is connected to the ground.

12. The ultrasonic atomizer according to claim 11, wherein the impedance branch further comprises a third inductor; and wherein:
a first end of the third inductor is connected to the first end of the third capacitor and the resonance branch separately, and a second end of the third inductor is connected to the ultrasonic atomization sheet; or
the first end of the third inductor is connected to the resonance branch, and the second end of the third inductor is connected to the first end of the third capacitor and the ultrasonic atomization sheet separately.

13. The ultrasonic atomizer according to any one of claims 1 to 12, wherein:
the impedance of the combination of the impedance branch and the ultrasonic atomization sheet comprises an impedance real part and an impedance imaginary part, and
when the impedance real part is equal to the impedance of the combination of the power supply branch, the switching branch, and the resonance branch, and the impedance imaginary part is zero, the impedance of the combination of the impedance branch and the ultrasonic atomization sheet matches the impedance of the combination of the power supply branch, the switching branch, and the resonance branch.

14. The ultrasonic atomizer according to claim 1, wherein:
the control circuit further comprises a driving branch;
the switching branch is connected to the controller through the driving branch;
the driving branch is connected to the power supply; and
the driving branch is used for receiving the first pulse signal, and outputting a second pulse signal to the switching branch based on the first pulse signal and the power supply, wherein a driving capability of the second pulse signal is stronger than that of the first pulse signal.

15. The ultrasonic atomizer according to claim 14, wherein:
the driving branch comprises a driving chip, and the driving chip comprises a power input end, at least one signal input end, and at least one signal output end;
the power input end is connected to the power supply;
the signal input end is connected to the controller;
the signal output end is connected to the switching branch;
the signal input end is used for inputting the first pulse signal; and
the signal output end is used for outputting the second pulse signal.

16. The ultrasonic atomizer according to claim 1, wherein:
the control circuit further comprises a current detection branch;
the current detection branch is connected to the power supply, the power supply branch, and the controller separately; and
the current detection branch is used for detecting a current flowing into the power supply branch.

17. The ultrasonic atomizer according to claim 16, wherein:
the current detection branch comprises an amplifier and a third resistor;
the third resistor is connected to the amplifier, the power supply branch, and the power supply separately;
the amplifier is connected to the controller; and
the amplifier is configured to output a detection voltage based on a voltage across the third resistor, to enable the controller to determine, based on the detection voltage, the current flowing into the power supply branch.
